# EUROPEAN PATENT APPLICATION

(11) **EP 1 371 352 A1**
(43) Date of publication of application: **17.12.2003**
(21) Application number: 02447118.7
(22) Date of filing: 13.06.2002
(51) Int. Cl.: A61K 7/06, A61P 17/14

(54) **Use of a herb mixture for stimulating and/or inducing hair growth**

(71) Applicant: De Laet, Hans, 2570 Duffel (BE)
(72) Inventor: De Laet, Hans, 2570 Duffel (BE)
(74) Representative: Van Reet, Joseph

(57) **Abstract**

The invention relates to the use of a herb mixture either as a cosmetic product for stimulating and/or inducing hair growth and/or for preventing or reducing hair losses, wherein an extract of the herb mixture in an organic solvent is applied to the scalp, or for the manufacture of a medicament for the treatment of diseases such as alopecia involving hair losses, wherein the medicament is made from the extract of the herb mixture in an organic solvent. The herb mixture comprises at least aloe, myrrh, senna leaves, camphor, rhubarb roots, manna, theriac venezian, carline thistle roots, angelica roots and zedoary roots.

## Description

The present invention relates to the use of a herb mixture comprising angelica roots as a cosmetic product for stimulating and/or inducing hair growth and/or for preventing or reducing hair losses, wherein an extract of the herb mixture is applied to the scalp. The invention further also relates to the use of a herb mixture comprising at least angelica roots for the manufacture of a medicament for the treatment of diseases such as alopecia involving hair losses, wherein the medicament is made from an extract of the herb mixture.

In practice extracts of herb mixtures including angelica roots are known which have an advantageous effect on the growth of hair. Such extracts are described for example in CN-A-1 081 613, CN-A-1 172 639 and GB-B-2 240 715. As described in GB-B-2 240 715, angelica in botanical terms denotes a large genus of tall, perennial herbs of the parsley family. The particular angelica which is believed to be of most interest in the context of the present invention is Angelica archangelica or Angelica officinalis.

An object of the present invention is to provide a new herb mixture which comprises angelica roots and which has an even better effect on the growth of hair or against hair losses.

In accordance with the present invention, the herb mixture further comprises, in addition to the angelica roots, at least aloe, myrrh, senna leaves, camphor, rhubarb roots, manna, theriac venezian, carline thistle roots and zedoary roots.

Tests have shown that a treatment of the scalp with an extract of this herb mixture stimulates and induces very clearly hair growth with people of different ages who suffer from an either or not strong degree of hair losses.

As to the herb mixture used in accordance with the present invention, it should be noted that this herb mixture is already known per se in practice under the denomination "Swedish Herbs" and the alcoholic extract thereof under the denomination "Swedish Bitters". The formula for this herb mixture was originated by Paracelsus in the 1500's. More recently, the Austrian herbalist Maria Treben described many benefits of the Swedish bitters for the human health in her book "Health Through God's Pharmacy". Apparently, there would be hardly any illness where Swedish herbs are not of benefit. However, notwithstanding the fact that the Swedish herbs have been used for about 400 years nobody has discovered hitherto its very beneficial effects on the restoration of hair growth.

In an advantageous embodiment of the use according to the present invention, the herb mixture is extracted in a water-alcohol mixture containing in particular at least 35 % alcohol, preferably at least about 40 % alcohol.

The alcohol is preferably ethanol. The water-alcohol mixture may be an artificial mixture or may for example be rye or fruit spirit having in particular an alcohol content of 38% to 40% or more.

In accordance with the invention, use is made in general of a herb mixture to stimulate and/or induce hair growth and/or to prevent or reduce hair losses either as a cosmetic product or as a medicine for treating diseases which involve the loss of hair. Examples of such diseases are alopecia aerata or chronic alopecia.

The herb mixture is not applied as such to the scalp but first an extract is made in an organic solvent. This solvent is preferably a water-alcohol mixture containing at least 35% alcohol and preferably at least about 40% alcohol or more. The alcohol will usually comprise ethanol. Use can be made of concentrated alcohol mixed with water or of a spirit such as rye or fruit spirit made by a fermentation process and having in particular an alcohol content of 38 to 40%. The alcoholic extract can be applied as such onto the scalp, more particularly onto the bald portions thereof or onto portions having a reduced number of hears. However, it is also possible to incorporate the extract into other topical application forms such as cream's, shampoo's, lotions, gels, etc. Before incorporating the extract in these application forms, the alcohol is preferably removed, in particular by evaporation.

The strength of the herbal extract depends on the time the herb mixture is kept in the solvent and also the amount of herb mixture. Usually, an amount of about 5 to about 7 parts by weight herb mixture will be added to 100 parts by weight of solvent. After the extraction, some of the solvent may be removed by evaporation. When use is made of a solvent consisting of a water-alcohol mixture, the alcohol is preferably evaporated.

The herb mixture used in accordance with the present invention comprises at least aloe, myrrh, senna leaves, camphor, rhubarb roots, manna, theriac venezian, carline thistle roots, angelica roots and zedoary roots. The theriac venezian is a well-known herb mixture, and available as such in the specialized stores. It comprises a blend of the following herbs and spices : angelica root, cimicifuga root, valerian root, cinnamon bark, zedoary roots, cardamon and myrrh. Instead of this preprepared mixture, use can of course also be made of the separate herbs.

The above-mentioned herbs are preferably used in the following amounts :
aloe : about 10 to about 14 parts by weight.
myrrh : about 4 to about 6 parts by weight.
senna leaves : about 8 to about 12 parts by weight.
camphor :about 8 to about 10 parts by weight.
rhubarb roots : about 8 to about 12 parts by weight.
manna : about 8 to about 10 parts by weight.
theriac venezian : about 8 to about 12 parts by weight.
carline thistle roots : about 4 to about 6 parts by weight.
angelica roots : about 8 to about 12 parts by weight.
zedoary roots : about 8 to about 12 parts by weight.

In addition to these herbs, the herb mixture may further contain other herbs or spices, in particular saffron. Saffron is preferably used in an amount of about 0 to about 0.4 parts by weight, although larger amounts, for example amounts of about 2 parts by weight as in the known "Swedish Herbs" can also be used.

Some of the herbs used in accordance with the present invention are already used in other herb mixtures for treating baldness, in particular the angelica roots. However, based on the literature about the "Swedish Herbs", one would not expect the surprising effects of this particular herb mixture on the hair growth. In her book "Health Through God's Pharmacy", Maria Treben describes indeed the different known properties of each of the herbs included in the "Swedish Herbs" but none of these properties suggests any effect of the combined herb mixture on the hair growth. Moreover, even from the numerous effects of the "Swedish Herbs" in the treatment of various diseases or illnesses reported by her, one would not expect the benefits of this mixture in the treatment of baldness. In fact, synergistic effects on the stimulation or induction of hair growth appear to arise by the particular combination of herbs.

The present inventor has done tests with the available "Swedish Herbs" extracted in 1.5 litre of 40 % rye spirit. He put more particularly 10 g aloe, 5 g myrrh, 2 g saffron, 10 g senna leaves 10 g camphor, 10 g rhubarb roots, 10 g manna, 10 g theria venezian, 5 g carline thistle roots, 10 g angelica roots and 10 g zedoary roots in a wide-necked 2 litre bottle and poured 1.5 litre of rye spirit thereover. The bottle was left standing in a heated living room for 2 to 3 weeks and shaken at least daily. Subsequently, the alcohol was removed by evaporation. The liquid was then strained and stored in a cool place. It was divided into single dosage units of for example 6 ml, in particular in ampoules or vials.

Some people suffering from premature hair losses were treated twice a day with 6 ml of the thus prepared herb extract. The herb extract was simply poured on the bald portions of the scalp and massaged in with the finger tips. The product penetrated thus quite quickly into the skin. For all of the thus treated people the hair growth recovered nearly completely after a treatment of 4 to 10 months.

Some other, older people of about 40 suffering from hair losses, were also treated in the same way. Depending on the degree of hair losses or baldness, they showed a recovery of their hair growth after a period of 4 to 12 months.

For still older people suffering from baldness, 6 ml of the prepared extract was also applied twice a day on the scalp. Most of these people showed new hair growth after such a treatment for a period of 4 to 12 months.

The first two tested groups of people showed already after a week or 4 new hairs having a length of 1 to 1.5 cm. After a period of 8 to 16 weeks, those new hairs reached already a length of 3 to 5 cm and more.

The third group of older people showed less quickly results. However, on a longer term, new hair growth could also be observed with these older people.

Further tests have shown that saffron is not an essential component of the herb mixture but may also have beneficial effects, in particular in amounts of 0 to 0.4 parts by weight, based on the parts by weight of the other ingredients described hereabove.

Other tests have shown that the herb extract according to the present invention has better effects than Regaine 2% minoxidil, which is nowadays known as the most effective hair restorer.

In summary, the herb extract used in accordance with the present invention, showed positive effects on the hair growth for all skin or hair colours, for young and older people and for men, women and even children.

It will be clear that the herb extract cannot only be applied as such to the skin, but can for example be combined with other excipients or carriers to obtain other topical application forms, for example ointments or even shampoo's, lotions, gels, etc. The present inventor has also been found that a portion of the herb mixture extract can be taken up orally in order to support the effect of the topical application form wherein the other portion of the herb mixture extract is applied. No side effects of the oral intake have been noticed.

## Claims

1. Use of a herb mixture comprising angelica roots as a cosmetic product for stimulating and/or inducing hair growth and/or for preventing or reducing hair losses, wherein an extract of the herb mixture is applied to the scalp, **characterised in that** said herb mixture further comprises at least aloe, myrrh, senna leaves, camphor, rhubarb roots, manna, theriac venezian, carline thistle roots and zedoary roots.

2. Use of a herb mixture comprising angelica roots for the manufacture of a medicament for the treatment of diseases such as alopecia involving hair losses, wherein the medicament is made from an extract of the herb mixture, **characterised in that** said herb mixture further comprises at least aloe, myrrh, senna leaves, camphor, rhubarb roots, manna, theriac venezian, carline thistle roots and zedoary roots.

3. Use according to claim 1 or 2, **characterised in that** the herb mixture comprises about 10 to about 14 parts by weight aloe, about 4 to about 6 parts by weight myrrh, about 8 to about 12 parts by weight senna leaves, about 8 to about 10 parts by weight camphor, about 8 to about 12 parts by weight rhubarb roots, about 8 to about 10 parts by weight manna, about 8 to about 12 parts by weight theriac venezian, about 4 to about 6 parts by weight carline thistle roots, about 8 to about 12 parts by weight angelica roots and about 8 to about 12 parts by weight zedoary roots.

4. Use according to claim 3, **characterised in that** the herb mixture comprises about 10 parts by weight aloe, about 5 parts by weight myrrh, about 10 parts by weight senna leaves, about 10 parts by weight camphor, about 10 parts by weight rhubarb roots, about 10 parts by weight manna, about 10 parts by weight theriac venezian, about 5 parts by weight carline thistle roots, about 10 parts by weight angelica roots and about 10 parts by weight zedoary roots.

5. Use according to any one of the claims 1 to 4, **characterised in that** the herb mixture further contains saffron.

6. Use according to claim 5, **characterised in that** the herb mixture comprises about 0 to about 0.4 parts by weight saffron.

7. Use according to any one of the claims 1 to 6, **characterised in that** the herb mixture is extracted in a water-alcohol mixture containing in particular at least 35 % alcohol, preferably at least about 40 % alcohol.

8. Use according to any one of the claims 1 to 7, **characterised in that** the herb mixture is extracted in a ratio of about 5 to about 7 parts by weight per 100 parts by weight of solvent.

9. Use according to any one of the claims 1 to 8, **characterised in that** a portion of the herb mixture extract is taken up orally.

10. Use according to any one of the claims 1 to 9, **characterised in that** the herb mixture extract is applied at least once a day, preferably at least twice a day.
